# EUROPÄISCHE PATENTANMELDUNG

(11) **EP 4 566 564 A1**
(43) Veröffentlichungstag der Anmeldung: **11.06.2025**
(21) Anmeldenummer: 24216489.5
(22) Anmeldetag: 29.11.2024
(51) Int. Cl.: A61B 90/70, A61L 2/24, A61L 2/025, A61L 2/07

(54) **VORRICHTUNG ZUR AUFBEREITUNG VON MEDIZINISCHEN INSTRUMENTEN**

(30) Priorität: 04.12.2023 AT 509702023
(71) Anmelder: s&m Verwaltungs-GmbH, 6900 Bregenz (AT)
(72) Erfinder: Bischof, Thomas, 6900 Bregenz (AT)
(74) Vertreter: Riebling, Peter

(57) **Zusammenfassung**

Vorrichtung (1) zur Aufbereitung von medizinischen Instrumenten, bestehend aus einem Hygieneraumgehäuse (16), in welchem eine Reinigungs- und Desinfektionsvorrichtung (13) angeordnet ist, welche als Ultraschallvorrichtung ausgebildet ist und eine Reinigung und Desinfektion der medizinischen Instrumente durchführt, dadurch gekennzeichnet, dass das Hygieneraumgehäuse (16) als ein gas- und druckdichtes Gehäuse ausgebildet ist, innerhalb welchem die Reinigungs- und Desinfektionsvorrichtung (13) und zusätzlich eine Sterilisationsvorrichtung (17) angeordnet ist, so dass innerhalb des Hygieneraumgehäuses (16) eine Reinigung, Desinfektion und Sterilisation der medizinischen Instrumente erfolgt.

## Beschreibung

Die Erfindung betrifft eine Vorrichtung zur Aufbereitung von medizinischen Instrumenten gemäß dem Oberbegriff des Patentanspruches 1 und des Patentanspruches 8.

Die Aufbereitung von Instrumenten in einer medizinischen bzw. zahnmedizinischen Praxis erfolgt meistens maschinell mit Hilfe eines Reinigungs- und Desinfektionsgerät (kurz: RDG-Geräte) und einem Sterilisator.

Medizinische Instrumente, welche lediglich mit der Haut bzw. Schleimhaut in Berührung kommen, werden nur gereinigt und desinfiziert. Meistens wird für die Desinfektion ein maschineller Thermodesinfektor eingesetzt, welcher bei einer Hitze von mindestens 90°C die Bakterien, Viren und Pilze abtötet. Alle anderen medizinischen Instrumente, welche im Rahmen von chirurgischen, parodontologischen oder endodontologischen Maßnahmen beim Patienten angewandt werden, müssen nach deren Verwendung (zusätzlich) sterilisiert werden. Die Sterilisation erfolgt mit Wasserdampf in einem Autoklav bei 134 °C, wobei sämtliche Formen von mikrobiellem Leben vernichtet werden, um eine absolute Keimfreiheit zu erreichen. Vor dem Sterilisationsvorganges werden die Instrumente keimdicht verpackt und stehen so nach der Sterilisation längere Zeit steril für eine erneute Behandlung zur Verfügung.

Mit der EP 4 151 544 A1 wird eine automatisierte Vorrichtung zur Sterilisation und Verpackung von zahnmedizinischen und/oder chirurgischen Instrumenten offenbart, wobei ein Roboter mit einem Greifer (Manipulator) einen Behälter mit Instrumenten zu einer Sterilisationsvorrichtung und einer Verpackungsvorrichtung bewegt. Die vorliegende Vorrichtung ist sehr aufwändig und kostenintensiv und ist nicht für den Einsatz in einem Behandlungsraum einer Praxis vorgesehen.

Mit der WO 2007/000639A1 wird ein Verfahren zur Sterilisation und Verpackung von chirurgischen Instrumenten offenbart. Bei dem Verfahren werden die einzelnen Instrumente in verschiedene Kammern weitergegeben, wobei in einer ersten Kammer eine Sterilisation und in einer weiteren Kammer eine Verpackung der sterilisierten Instrumente durchgeführt wird. Für das vorliegende Verfahren benötigt man einen sehr großen Raum, welcher in einer Zahnarztpraxis nicht zur Verfügung steht.

Mit der Druckschrift US 2021/0236676 A1 wird eine Vorrichtung zum Bearbeiten von medizinischen Instrumenten offenbart. Die Vorrichtung besteht aus einer linear verfahrbaren Handhabungsvorrichtung, welche Trays mit zu reinigenden Instrumenten von einer Station zur nächsten Station bewegt. Die erste Station ist ein Art Tauchbad, in welches die schmutzigen Instrumente mit Hilfe der Handhabungsvorrichtung eingetaucht werden. Im Anschluss werden die vorgereinigten Instrumente von der Handhabungsvorrichtung wieder entnommen und an eine Sterilisationsvorrichtung übergeben. Nach der erfolgten Sterilisation werden die Instrumente getrocknet und verpackt. Auch bei dieser Vorrichtung besteht der Nachteil, dass die Vorrichtung aufgrund der linear verfahrbaren Handhabungsvorrichtung sehr groß ist.

Ausgehend von den bekannten Vorrichtungen zur Reinigung, Desinfektion und Sterilisation von chirurgischen und (zahn-)medizinischen Instrumenten besteht nun die Aufgabe darin, eine Vorrichtung bereitzustellen, welche klein genug ist, sodass sie innerhalb eines Behandlungszimmers in einer medizinischen Praxis eingesetzt werden kann und trotzdem eine fachgerechte Reinigung, Desinfektion und Sterilisation der Instrumente erreicht wird.

Die Lösung der gestellten Aufgabe wird durch die wesentlichen Merkmale des Anspruches 1 und des Anspruches 8 gekennzeichnet.

Wesentliches Merkmal ist, dass die erfindungsgemäße Vorrichtung ein Hygieneraumgehäuse (16) aufweist, welches als ein gas- und druckdichtes Gehäuse ausgebildet ist, innerhalb welchem die Reinigungs- und Desinfektionsvorrichtung (13) und zusätzlich eine Sterilisationsvorrichtung (17) angeordnet ist, wobei innerhalb des Hygieneraumgehäuses (16) eine Reinigung, Desinfektion und Sterilisation der medizinischen Instrumente erfolgt.

Mit der gegebenen technischen Lehre ergibt sich der wesentliche Vorteil, dass mit nur einem abgeschlossenen Gerät (Vorrichtung), welches relativ klein ausgebildet ist, eine automatisierte vollständige Instrumentenaufbereitung von medizinischen Instrumenten in einem Behandlungsraum einer Praxis durchgeführt werden kann.

Im Folgenden wird der Begriff medizinische Instrumente als Sammelbegriff für medizinische und/oder zahnmedizinische Instrumente verwendet. Dies können ebenfalls Trays, Aufbewahrungsbehälter oder dergleichen sein, welche im Rahmen einer Behandlungspraxis eingesetzt und aufbereitet werden müssen. Die erfindungsgemäße Vorrichtung ist so ausgebildet, dass die medizinischen Instrumente entweder alleine oder auf einem Tray gereinigt, desinfiziert und/oder sterilisiert werden können.

Unter einem Tray wird ein offenes Verpackungsmittel verstanden. Dies kann beispielsweise ein Tablett, eine Ablage, ein Behälter, eine Schachtel oder dergleichen sein. Das Tray besteht beispielsweise aus einem Kunststoff oder Metallmaterial. Auf der Oberseite weist das Tray vorzugsweise Vorrichtungen zum Einklemmen der zu reinigenden, medizinischen Instrumente auf. Eine Klemmung der Instrumente ist vorteilhaft, da dann sichergestellt ist, dass während des Reinigungsvorganges keine Instrumente übereinanderliegen. Es ist jedoch auch möglich, dass die Instrumente nur auf das Tablett gelegt werden und somit eben keine Festlegung der Instrumente gegenüber dem Tray stattfindet.

Bei einer ersten bevorzugten Ausführungsform weist die Vorrichtung ein gas- und druckdicht abgeschlossenes Gehäuse auf, welches den eigentlichen Hygieneraum bildet. Das Gehäuse des Hygieneraums wird daher als Hygieneraumgehäuse bezeichnet. In dem Hygieneraumgehäuse läuft der Aufbereitungszyklus der medizinischen Instrumente ab, wobei mit einer Reinigungs- und Desinfektionsvorrichtung die Reinigung und Desinfektion der medizinischen Instrumente und mit einer Sterilisationsvorrichtung die Sterilisation durchgeführt wird. Die Abmessung des Gehäuses ist so groß, dass es in einem Behandlungsraum einer Arztpraxis aufstellbar ist.

Das abgeschlossene Hygieneraumgehäuse weist an mindestens einer Seite eine verschließbare Öffnung in Form einer Klappe auf, durch welche die zu reinigenden medizinischen Instrumenten, vorzugsweise auf einem Tray, in das Gehäuse eingeschoben werden. Die Klappe ist gas- und druckdicht ausgebildet, sodass innerhalb des Hygieneraums ein Reinraum besteht. Des Weiteren ist die Klappe thermisch isoliert.

Vorzugsweise befindet sich hinter der Klappe innerhalb des Gehäuses ein Förderband. Das Förderband übernimmt die medizinischen Instrumente bzw. das Tray mit den medizinischen Instrumenten und bewegt die vorgenannten Gegenstände innerhalb des Gehäuses in horizontaler Richtung. Mit Hilfe des Förderbandes wird das Tray über der Reinigungs- und Desinfektionsvorrichtung positioniert.

Vorzugsweise ist die Reinigungs- und Desinfektionsvorrichtung als Ultraschallgerät ausgebildet, wobei das Ultraschallgerät eine Ultraschallreinigung der medizinischen Instrumente durchführt. Das Ultraschallgerät befindet sich unterhalb des Förderbandes, wobei es eine nach oben offene Wanne aufweist, an deren Bodenbereich mehrere Ultraschallköpfe angeordnet sind. Innerhalb der Wanne befindet sich eine Flüssigkeit (z.B. Wasser mit oder ohne Reinigungs- bzw. Desinfektionsmittel), welches von den Ultraschallköpfen mit einer Ultraschall-Schwingung angeregt wird. Die Flüssigkeit innerhalb der Wanne wird zum Reinigungsvorgang mit einem Heizvorrichtung erhitzt. Vorzugsweise erfolgt eine Erwärmung auf mindestens 90 Grad, denn bei dieser Temperatur werden alle Bakterien, Viren und Pilze abgetötet.

Die Wanne mit den Ultraschallköpfen ist innerhalb des Gehäuses vertikal bewegbar. Für den Reinigungs- und Desinfektionsvorgang erfolgt eine vertikale Bewegung der Ultraschallvorrichtung in Richtung des Förderbandes, auf welchem sich das Tray mit den zu reinigenden medizinischen Instrumenten befindet, sodass die Flüssigkeit des Ultraschallbades mit den medizinischen Instrumenten in Kontakt kommt. Die Wanne des Ultraschallbades ist vorzugsweise so ausgebildet, dass die Flüssigkeit innerhalb der Wanne vollkommen den Bereich des Förderbandes und den darauf befindlichen Tray umschließt, sodass eine vollständige Reinigung und Desinfektion stattfinden.

Nach der erfolgten Reinigung und Desinfektion wird das Ultraschallbad wieder in vertikaler Richtung nach unten gefahren. Im Anschluss daran transportiert das Förderband das Tray mit den gereinigten, medizinischen Instrumenten nach Trocknung mit Druckluft in Richtung der Klappe des Gehäuses.

Die vertikale Bewegung der Ultraschallvorrichtung erfolgt mit einem Antrieb. Der Antrieb kann beispielsweise ein elektrischer Motor, ein elektrischer Linearantrieb oder ein Spindelhubantrieb sein. Es ist jedoch auch ein pneumatischer Antrieb möglich, wobei die vorhandene Druckluftquelle der Behandlungspraxis genutzt werden kann.

Die Reinigungs- und Desinfektionsvorrichtung kann statt als Ultraschallgerät auch als eine Art Spülmaschine ausgebildet sein, welche mit Hochdruck-Wasserdüsen die medizinischen Instrumente reinigt.

Das Gehäuse des Hygieneraums ist relativ klein ausgebildet. Beispielsweise weist das Gehäuse Abmessungen von 30 cm x 30 cm x 45 cm auf, so dass es ohne Weiteres in einem Behandlungsraum einer Praxis eingesetzt werden kann. Dies stellt einen wesentlichen Unterschied gegenüber dem Stand der Technik dar, welcher relativ große Vorrichtungen vorsieht, innerhalb welchen aufwendige Roboterarme und dergleichen angeordnet sind. Die Abmessungen des Hygieneraums sind jedoch nicht auf die oben genannten Abmessungen beschränkt, es ist durchaus möglich, dass der Hygieneraum auch größer ausgebildet ist.

Die Form des Hygieneraumgehäuse kann beispielsweise rund oder oval sein. Durch die runde Form kann der Druck besser von dem Gehäuse aufgenommen werden. Es sind jedoch auch andere geometrische Formen möglich.

Im Hygieneraum findet neben der Reinigung und Desinfektion mit der Reinigungs- und Desinfektionsvorrichtung auch eine Sterilisation mit einer Sterilisationsvorrichtung statt. Die Sterilisationsvorrichtung ist hierfür als Autoklav ausgebildet, welcher innerhalb des Hygieneraumgehäuse eine thermische Behandlung der medizinischen Instrumente im Überdruckbereich durchführt. Bei dem Hygieneraumgehäuse handelt es sich somit um einen Druckbehälter, welcher mit einer Klappe gas- und druckdicht verschließbar ist. Mit dem Autoklav wird beispielsweise ein Vakuumverfahren (B-Klasse) durchgeführt, wobei durch mehrmaliges Evakuieren die Luft innerhalb des Gehäuses entfernt wird und gleichzeitig im Wechsel Dampfeinströmungen erfolgen. Das Gehäuse des Hygieneraums muss dergestalt ausgebildet sein, dass es hohen Drücken und hohen Temperaturen standhält.

Mit der erfindungsgemäßen Vorrichtung findet somit in dem gleichen Gehäuse (Hygieneraumgehäuse) nicht nur eine Reinigung und Desinfektion der medizinischen Instrumente statt, sondern auch eine Sterilisation. Während beim Stand der Technik diese Verfahrensschritte auf unterschiedliche Räume und Vorrichtungen aufgeteilt gewesen ist, finden bei der erfindungsgemäßen Vorrichtung diese Verfahrensschritte alle in einem Gehäuse statt.

Mit der erfindungsgemäßen Vorrichtung ist es ferner möglich, auch einzelne Verfahrensschritte innerhalb des Hygieneraums separat anzuwenden. So kann beispielsweise innerhalb des Hygieneraums auch nur eine Reinigung und Desinfektion der Instrumente stattfinden und danach das Tray wieder aus Hygieneraum entnommen werden. Es ist somit nicht zwangsläufig, dass bei jeder Behandlung der Instrumente auch eine Autoklav-B-Sterilisation durchgeführt wird.

Bei dem Förderband, welches für den Transport des Trays innerhalb des Gehäuses eingesetzt wird, handelt es sich um ein angetriebenes Förderband. Wichtig ist, dass das eingesetzte Förderband für die Temperaturen und hohen Drücke ausgelegt ist.

Nach der durchgeführten Reinigung und Desinfektion muss das Tray mit den darauf befindlichen Instrumenten für eine Sterilisation verpackt werden. Die Verpackung des Trays erfolgt entweder händisch oder mit einer eigens hierfür vorgesehenen Vorrichtung. Im Anschluss daran wird das verpackte Tray erneut in den Hygieneraum hineingeschoben, um das verpackte Tray mit der Sterilisationsvorrichtung (Autoklav B) zu sterilisieren.

Die erfindungsgemäße Vorrichtung kann ferner gemäß dem nebengeordneten Anspruch 8 ein abgeschlossenes Gehäuse aufweisen, wobei innerhalb des abgeschlossenen Gehäuses ein gas- und druckdichtes Hygieneraumgehäuse angeordnet ist, in welchem sich eine Reinigungs- und Desinfektionsvorrichtung und eine Sterilisationsvorrichtung befinden und dass sich zusätzlich in dem abgeschlossenen Gehäuse eine Auf- und Nachbearbeitungsvorrichtung für die medizinischen Instrumente befindet.

Unter einer Auf- und Nachbearbeitungsvorrichtung versteht die vorliegende Erfindung eine Vorrichtung, mit welcher zunächst die schmutzigen Instrumente auf dem Tray digital erfasst werden, um eine erste Aufnahme bzw. Feststellung der Instrumente durchzuführen. Im Anschluss daran wird das Tray mit den Instrumenten in den Hygieneraum weiterbefördert, wo der eigentliche Reinigungs- und Desinfizierungsvorgang stattfindet. Nach dem Reinigungs- und Desinfektionsvorgang und vor einem fakultativen Sterilisationsvorgang wird in der Auf- und Nachbearbeitungsvorrichtung das Tray mit den darauf befindlichen medizinischen Instrumenten verpackt. Hierfür weist die Auf- und Nachbearbeitungsvorrichtung eine Folienverpackungsvorrichtung auf. Die Folienverpackungsvorrichtung ist beispielsweise als Folienschweißgerät ausgebildet, welches zwei Folien miteinander verschweißt, sodass eine keimdichte Verpackung des Trays erreicht wird.

Nachdem das Tray mit dem Folienschweißgerät verpackt wurde, wird es mit dem Förderband in den Hygieneraum transportiert, um eine Sterilisation des verpackten Trays durchzuführen. Im Anschluss daran wird das sterilisierte Tray wieder aus dem Hygieneraum herausgefahren. Vorteilhafterweise weist die Auf- und Nachbereitungsvorrichtung einen Drucker für die Kennzeichnung des sterilisierten Trays mit den medizinischen Instrumenten und die durchgeführten Prozesse im Hygieneraum auf. Der Drucker bedruckt ein Label, das auf die Folie aufgeklebt wird oder druckt direkt auf die Folie. Als Ergebnis erhält man allen hygienischen Vorgaben entsprechend aufbereitete medizinische Instrumente, welche in einer Folienverpackung keimdicht verpackt und gekennzeichnet sind.

Bei einer bevorzugten Ausführungsform befindet sich innerhalb des Gehäuses noch eine UV-Vorrichtung, wobei mit Hilfe von UV-Licht eine ständige Desinfizierung, insbesondere im Bereich der Auf- und Nachbearbeitungsvorrichtung, erfolgt.

Mit der erfindungsgemäßen Vorrichtung ist es somit möglich, innerhalb eines Gehäuses eine Bestandsaufnahme der medizinischen Instrumente, eine Reinigung, Desinfektion und Sterilisation, sowie eine Verpackung und Kennzeichnung durchzuführen. Die Prozesse laufen vorzugsweise automatisiert ab, wobei das Gerät eine sehr kleine Bauform aufweist, sodass es innerhalb eines Behandlungsraumes aufgestellt oder eingebaut werden kann.

Ein weiterer Vorteil der erfindungsgemäßen Vorrichtung ist, dass medizinische Instrumente eingespart werden können, da während der Behandlungszeit eines Patienten schon parallel hierzu eine Reinigung, Desinfektion und Sterilisation der beim vorigen Patienten benutzten Instrumente durchgeführt wird. So kann während der Behandlung eines ersten Patienten bereits die Reinigung der Instrumente für den nächsten Patienten durchgeführt werden.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von mehrere Ausführungswege darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Darstellung des erfindungsgemäßen Hygieneraums mit heruntergefahrenem Ultraschallbad;
- Figur 2:: Darstellung des erfindungsgemäßen Hygieneraums mit hochgefahrenem Ultraschallbad;
- Figur 3:: Perspektivische Darstellung der erfindungsgemäßen Vorrichtung;
- Figur 4:: Symbolische Darstellung der erfindungsgemäßen Vorrichtung mit dem Hygieneraum, der Auf- und Nachbearbeitungsvorrichtung sowie dem Technikraum;
- Figur 5:: Draufsicht auf die erfindungsgemäße Vorrichtung;
- Figur 6:: Seitenansicht auf die Auf- und Nachbearbeitungsvorrichtung;
- Figur 7:: Seitenansicht der Auf- und Nachbearbeitungsvorrichtung mit einem Tray, welches von einer Folie umgeben ist;
- Figur 8:: Seitenansicht auf die Auf- und Nachbearbeitungsvorrichtung mit Darstellung des Verschweißvorganges der beiden Folien;
- Figur 9:: Seitenansicht des Hygieneraums mit verpacktem, innenliegendem Tray beim Sterilisationsvorgang;

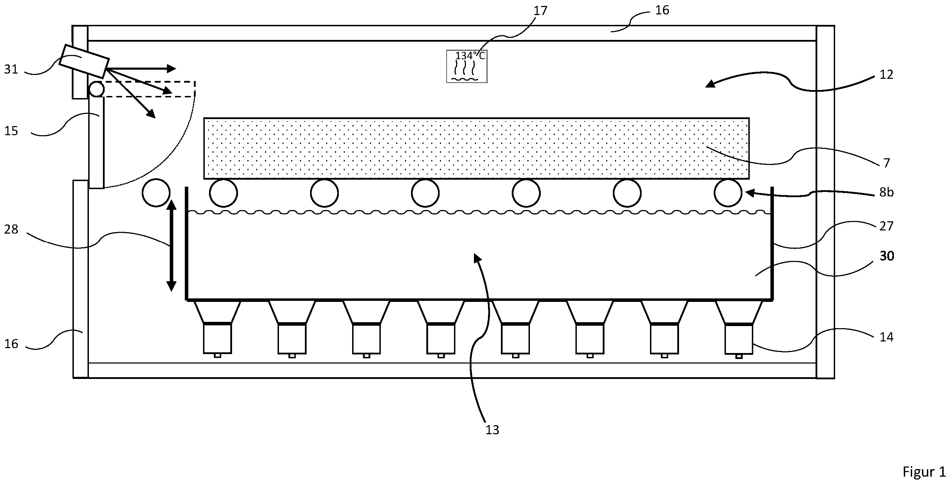

Mit der Figur 1 wird eine Vorrichtung zur Reinigung, Desinfektion und/oder Sterilisation von medizinischen Instrumenten gezeigt. Die Vorrichtung besteht aus einem Hygieneraum 12, welcher von einem abgeschlossenen Hygieneraumgehäuse 16 umgeben ist. Das Hygieneraumgehäuse 16 weist an mindestens einer Seitenwand eine gas- und druckdichte Klappe 15 auf, über welche die aufzubereitenden Instrumente in das Innere des Hygieneraumgehäuse 16 gelangen. Die Instrumente befinden sich auf einem Tray 7, welches durch die geöffnete Klappe 15 auf ein innenliegendes Förderband 8, 8b geschoben wird. Das Förderband 8, 8b ist angetrieben ausgebildet und bewegt das Tray 7 auf einer Ebene innerhalb des Hygieneraumgehäuses 16.

Unterhalb des Förderbandes 8, 8b befindet sich eine Reinigungs- und Desinfektionsvorrichtung 13. Die Reinigungs- und Desinfektionsvorrichtung 13 ist als Ultraschallvorrichtung ausgebildet und besteht im Wesentlichen aus einer Wanne 27 mit Ultraschallköpfen 14, welche am Boden der Wanne 27 angeordnet sind. Die Reinigungs- und Desinfektionsvorrichtung 13 ist in vertikaler Pfeilrichtung 28 beweglich. Gemäß der Figur 1 befindet sich die Reinigungs- und Desinfektionsvorrichtung 13 in der unteren, vertikalen Position. Durch eine vertikale Bewegung wird die Wanne 27 in Pfeilrichtung 28 nach oben zu dem Tray 7 gefahren, so dass die in der Wanne 27 befindliche Flüssigkeit 30 mit dem Förderband 8 und dem darauf befindlichem Tray 7 in Kontakt ist. Sobald die Flüssigkeit 30 den Tray 7 vollständig umgibt, können die Ultraschallköpfe 14 aktiviert werden. Nach dem erfolgten Reinigungsvorgang wird die Wanne 27 wieder nach unten d.h. weg von dem Tray 7 gefahren.

Durch die vertikale Verschiebung der gesamten Reinigungs- und Desinfektionsvorrichtung 13 bzw. der Wanne 27 mit den Ultraschallköpfen 14 ist es möglich, den gesamten Hygieneraum 12 relativ klein auszubilden, da nun keine eigene Kammer für die Ultraschallvorrichtung notwendig ist. Dies wird insbesondere durch die vertikale Verschiebbarkeit der gesamten Reinigungs- und Desinfektionsvorrichtung 13 innerhalb des Hygieneraumgehäuses 16 erreicht. Vorzugsweise erfolgt die vertikale Bewegung der Reinigungs- und Desinfektionsvorrichtung 13 mit elektrischen Antrieben.

Die Größe der Wanne 27 ist so ausgebildet, dass bei einer hochgefahrenen Wanne 27 der gesamte Tray 7 und gleichzeitig das Förderband 8 sich innerhalb der Wanne 27 befinden und von der darin befindlichen Flüssigkeit 30 bedeckt sind.

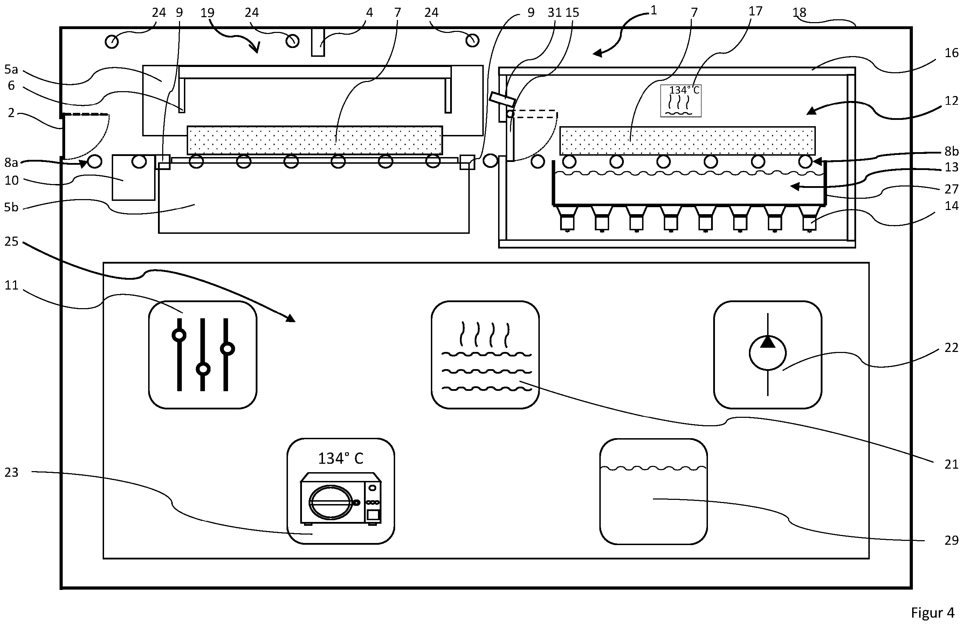

Bei der Flüssigkeit 30 handelt es sich beispielsweise um Wasser. Es sind jedoch auch andere Flüssigkeiten, wie beispielsweise eine Desinfektionsflüssigkeit möglich. Die Flüssigkeit 30 wird in einem Tank gespeichert, welcher sich unterhalb des Hygieneraums 12 befindet. Ein Austausch der Flüssigkeit 30 findet mit einer Pumpe 22 statt, auf welche in der Figur 4 näher eingegangen wird.

Um eine Desinfizierung des Trays 7 mit den darauf befindlichen Instrumenten zu erreichen, wird die Flüssigkeit 30 auf mindestens 90 Grad erhitzt. Hierfür ist eine eigens vorgesehene Heizvorrichtung 21 vorgesehen, auf welchen ebenfalls in der Figur 4 näher eingegangen wird.

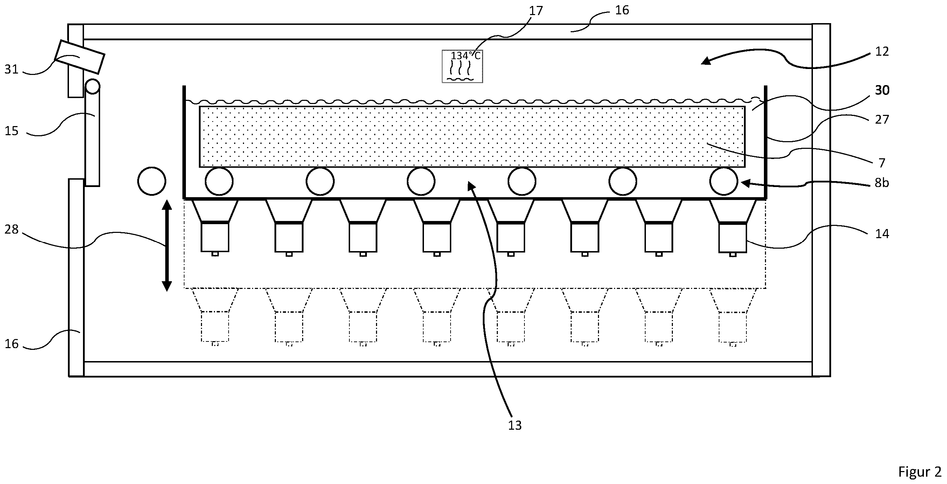

Mit der Figur 2 wird die Reinigungs- und Desinfektionsvorrichtung 13 in der oberen, vertikalen Position gezeigt. Die Flüssigkeit 30 in der Ultraschallwanne 27 bedeckt vollkommen das Tray 7 sowie das Förderband 8. Der Reinigungs- und Desinfizierungsvorgang ist durchführbar.

Nach dem Reinigungs- und Desinfizierungsvorgang wird die Ultraschallbadwanne 27 wieder nach unten gefahren. Das gereinigte und desinfizierte Tray 7 liegt weiterhin auf dem Förderband 8, 8b auf, wobei die Teile noch eine gewisse Restfeuchtigkeit aufweisen. Um eine Trocknung des Trays 7 durchzuführen, weist das Hygieneraumgehäuse 16 eine Druckluftquelle 31 auf, über welche Druckluft in das Gehäuse 16 eingeblasen wird. Mit Hilfe der Druckluft findet eine Trocknung des Trays 7 statt, sodass dieses mit nur einer geringen Restfeuchte wieder aus dem Hygieneraumgehäuse 16 kommt. Vorzugweise ist die Vorrichtung 1 mit einem allgemeinen Druckluftanschluss verbunden, welcher in jeder medizinischen Praxis zur Verfügung steht. Die Druckluft kann aber auch in der Vorrichtung 1 selbst erzeugt werden

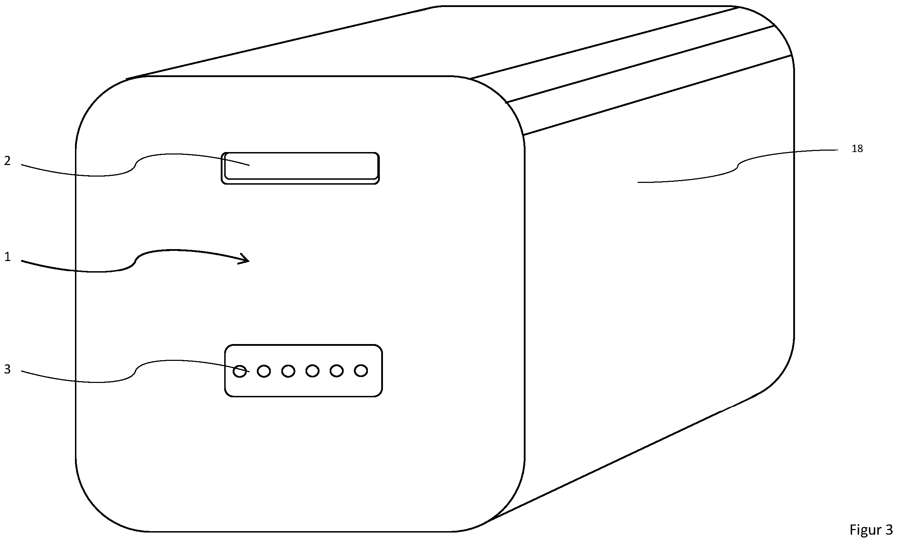

Mit der Figur 3 wird die Vorrichtung 1 gezeigt, welche im Wesentlichen aus einem geschlossenen Gehäuse 18 besteht, wobei über eine Klappe 2 die zu reinigenden Trays 7 eingeschoben und wieder entnommen werden. Des Weiteren weist die Vorrichtung 1 ein außenliegendes Bedienelement 3 auf, mit welchem der Reinigungs- und Desinfektionsvorgang gesteuert wird.

Die Figur 4 zeigt eine weitere Ausführungsform der erfindungsgemäßen Vorrichtung 1, welche aus einem geschlossenen Gehäuse 18 besteht, in welchem sich das Hygieneraumgehäuse 16 mit der Reinigungs- und Desinfektionsvorrichtung 13 und der Sterilisationsvorrichtung 17, sowie die Auf- und Nachbearbeitungsvorrichtung 19 befinden. Unterhalb des Gehäuses 18 befindet sich ferner ein Technikraum 25. Der Technikraum 25 kann dem Gehäuse 18 auch nur zugeordnet sein, was bedeutet, dass sich der Technikraum 25 an einer anderen örtlichen Stelle befindet und über diverse Verbindungen mit dem Gehäuse 18 verbunden ist.

Das Tray 7 mit den darauf befindlichen zu reinigenden Instrumenten wird zunächst über die Klappe 2 in das Gehäuse 18 auf ein erstes Förderband 8, 8a geschoben. nach der Klappe 2 befindet sich zunächst die Auf- und Nachbearbeitungsvorrichtung 19. Die Auf- und Nachbearbeitungsvorrichtung 19, weist eine Kamera 4 auf, mit welcher eine bildliche Erfassung der zu reinigenden Instrumente auf dem Tray 7 erfolgt. Aufgrund der bildlichen Erfassung wird der Reinigungs- Desinfektion- und Sterilisationsprozess je nach Instrumentenbelegung automatisch gesteuert. Im Anschluss daran wird mit Hilfe des Förderbandes 8, 8a das Tray 7 in den Hygieneraum 12 befördert. Der Hygieneraum 12 besteht aus einem Hygieneraumgehäuse 16 und weist eine eigene gas- und druckdichte Klappe 15 auf. Innerhalb des Hygieneraums 12 befindet sich ebenfalls ein zweites Förderband 8, 8b, mit welchem das Tray 7 bewegt wird. Vorzugsweise ist das erste Förderband 8a mit dem zweiten Förderband 8b verbunden. Das Hygieneraumgehäuse 16 bildet eine eigene abgeschlossene Einheit innerhalb des Gehäuses 18 der Vorrichtung 1 aus.

nach erfolgter Reinigung und Desinfizierung wird das Tray 7 mit Hilfe von Druckluft (Druckluftquelle 31) getrocknet und über die Klappe 15 aus dem Hygieneraum 12 herausgefahren. Das Förderband 8, 8a übernimmt das Tray 7 und positioniert es in der Auf- und Nachbearbeitungsvorrichtung 19. Die Auf- und Nachbearbeitungsvorrichtung 19 weist eine Folienverpackungsvorrichtung auf, welche als Folienschweißgerät 6 ausgebildet ist. Die Folienverpackungsvorrichtung weist ferner ein Folienförderband 9 auf, mit welchen die nach 5a und 5b um das Tray 7 gelegt und dann mit Hilfe des Folienschweißgerätes 6 verbunden werden. Als Ergebnis erhält man das Tray 7 mit gereinigten und desinfizierten Instrumenten, welche mit der Folie 5 keimdicht verpackt ist.

Die Vor- und Nachbereitungsvorrichtung 19 wird mit UV-Lampen 24 desinfiziert, welche über der Auf- und Nachbearbeitungsvorrichtung 19 angeordnet sind.

nach Abschluss des Verpackungsvorgangs wird das Tray 7 erneut mit dem Förderband 8 in den Hygieneraum 12 befördert und dort mit Hilfe einer Sterilisation-Vorrichtung 17 sterilisiert. Die Sterilisation-Vorrichtung 17 ist als Autoklav-B ausgebildet, welcher bei hohen Drücken und hohen Temperaturen eine Sterilisation durchführt. Das Hygieneraumgehäuse 16 muss daher für diese hohen Drücke und Temperaturen ausgebildet sein. In diesem Hygieneraumgehäuse 16 werden somit die medizinischen Instrumente gereinigt, desinfiziert und sterilisiert.

nach der erfolgten Sterilisation kann nun das in Folie 5 eingepackte Tray 7 wieder über die Klappe 15 aus dem Hygieneraumgehäuse 16 ausgefahren werden. Im Anschluss führt die Auf- und Nachbearbeitungsvorrichtung 19 eine Kennzeichnung des Trays 7 beispielsweise mit einem Label aus. Hierfür ist ein Drucker 10 vorgesehen, welcher die erforderlichen Daten des hygienischen Aufbereitungsvorganges beispielsweise auf ein Label 7 aufdruckt und auf die Folie klebt.

Gemäß der Figur 4 befindet sich unterhalb der Auf- und Nachbearbeitungsvorrichtung 19 und dem Hygieneraumgehäuse 16 ein Technikraum 25. Der Technikraum 25 weist alle Vorrichtungen bzw. Betriebseinheiten auf, welche für den Reinigungs- Desinfektions- und Sterilisationsprozess notwendig sind.

Im Technikraum 25 befindet sich eine Steuerung 11, mit welcher die Vorrichtungen gesteuert werden. Mit der Steuerung 11 findet beispielsweise eine Steuerung des Förderbandes 8, eine Steuerung des Öffnungs- und Schließvorgang der Klappen 2 und 15, eine Steuerung der vertikalen Bewegung der Reinigungs- und Desinfektionsvorrichtung 13, eine Steuerung der Reinigungs- und Desinfektionsvorrichtung 13, eine Steuerung des Druckluftanschlusses 31, eine Steuerung des Folienschweißgerätes 6, eine Steuerung der Zuführvorrichtung der Folie 5, eine Steuerung für den Heißdampferzeuger 23 für die Sterilisationsvorrichtung 17, eine Steuerung der Kamera 4, eine Steuerung der UV-Lampen 24, sowie eine Steuerung des Druckers 10 statt.

Im Technikraum 25 befinden sich ferner die Heizvorrichtung 21, mit welchem die Flüssigkeit 30 erhitzt wird, die Pumpe 22, mit welcher die Flüssigkeit 30 ausgehend von einem Tank 29 gefördert wird und der Heißdampferzeuger 23 für die Sterilisationsvorrichtung.

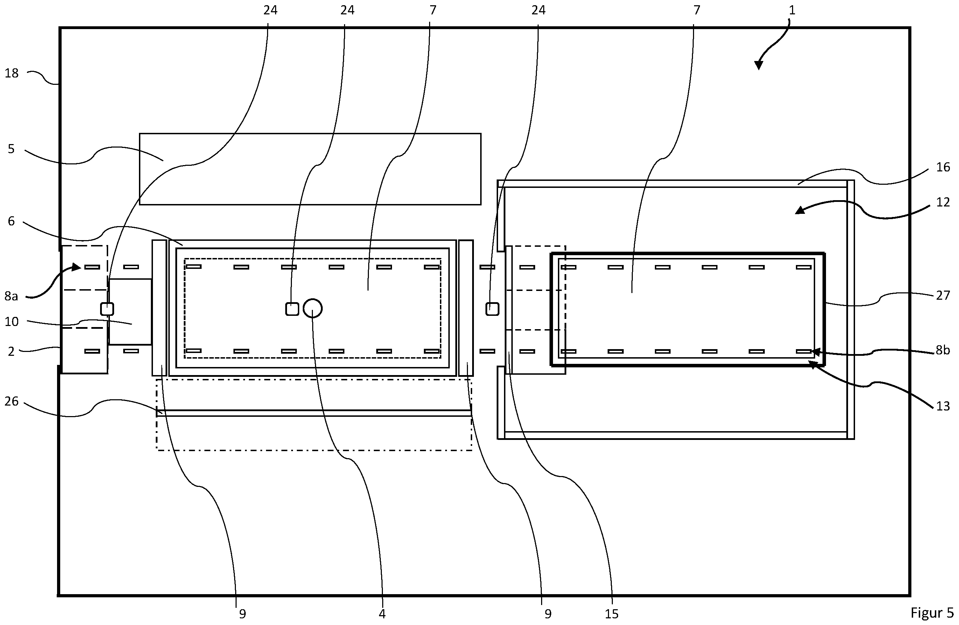

Figur 5 zeigt eine Draufsicht auf die Vorrichtung 1. Die Vorrichtung 1 weist ein Gehäuse 18 auf, innerhalb welchem die Auf- und Nachbearbeitungsvorrichtung 19 sowie das Gehäuse 16 des Hygieneraums 12 angeordnet sind. Oberhalb der Auf- und Nachbearbeitungsvorrichtung 19 befinden sich die UV-Lampen 24 sowie die Kamera 4. Des Weiteren ist erkennbar, dass von der Folie 5, mit welcher das Tray 7 eingepackt wird, eine Restfolie 26 im seitlichen Bereich des Trays 7 übrigbleibt, welche von einer dafür vorgesehenen Vorrichtung wiederaufgenommen wird.

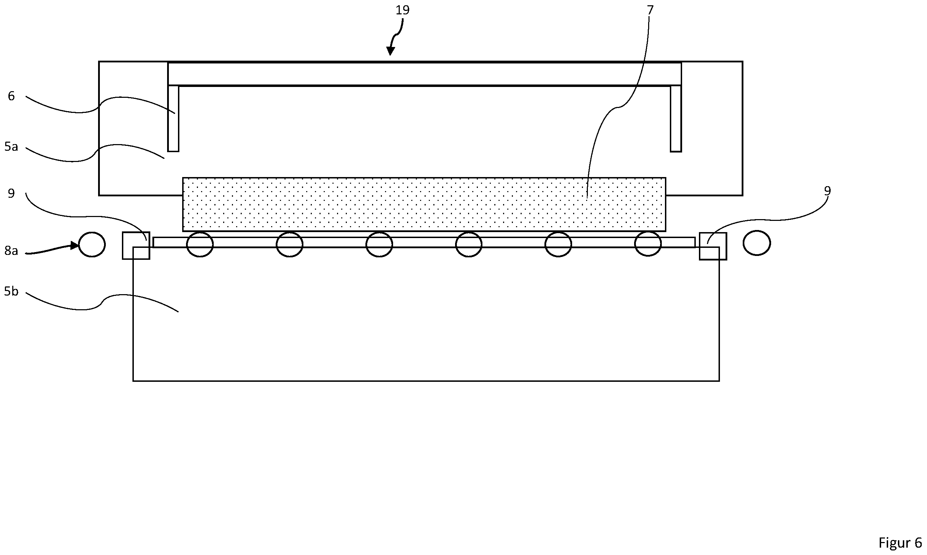

Mit der Figur 6 wird die Auf- und Nachbearbeitungsvorrichtung 19 gezeigt. Auf dem Förderband 8 befindet sich ein Tray 7 mit gereinigten Instrumenten.

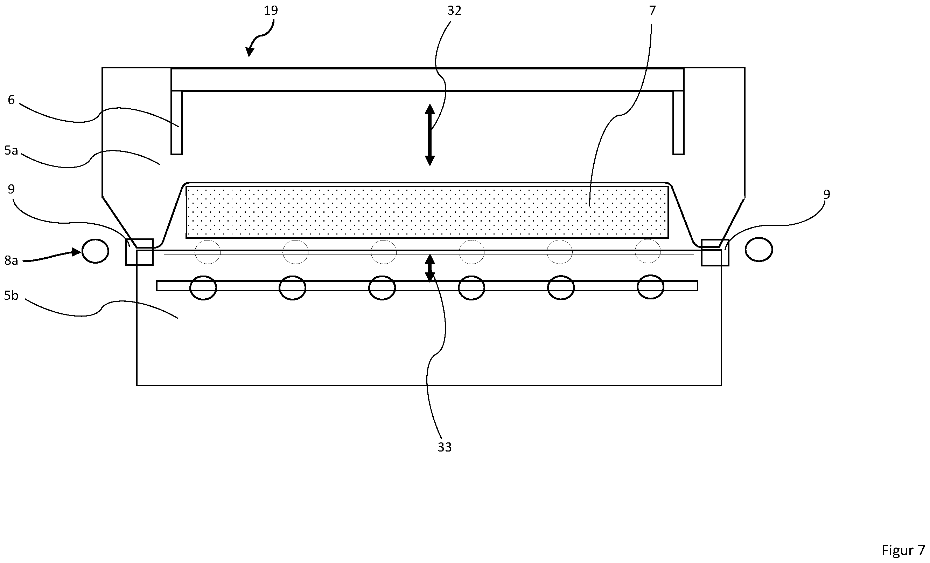

Mit der Figur 7 wird der Verpackungsvorgang des Trays 7 mit dem Folienschweißgerät 6 gezeigt. Das Folienschweißgerät 6 ist in Pfeilrichtung 32 vertikal bewegbar und kann dadurch in Richtung des Trays 7 gefahren und wieder von dem Tray 7 weggefahren werden. Mit dem Folienschweißgerät 6 werden die beiden Folien 5a und 5b verbunden, welche sich oberhalb und unterhalb des Trays 7 befinden. Nach dem erfolgten Schweißvorgang bleibt eine Restfolie 26 (Figur 5) übrig, welche von einer eigenen Vorrichtung wiederaufgenommen wird.

Um die beiden Folien 5a und 5b ordnungsgemäß zu verschweißen, ist das Förderband 8 ebenfalls vertikal beweglich innerhalb der Auf- und Nachbearbeitungsvorrichtung 19 angeordnet. Bei einer bevorzugten Ausführungsform wird bei einem Herunterfahren des Förderbandes 8 das Tray 7 von vier beweglichen Zapfen während des Folieneinziehvorganges gehalten. Nachdem die Folie 5 eingezogen ist, wird das Förderband 8 wieder in vertikaler Richtung nach oben gefahren, sodass dieses mit der Folie 5b und dem darauf befindlichen Tray 7 in Kontakt ist.

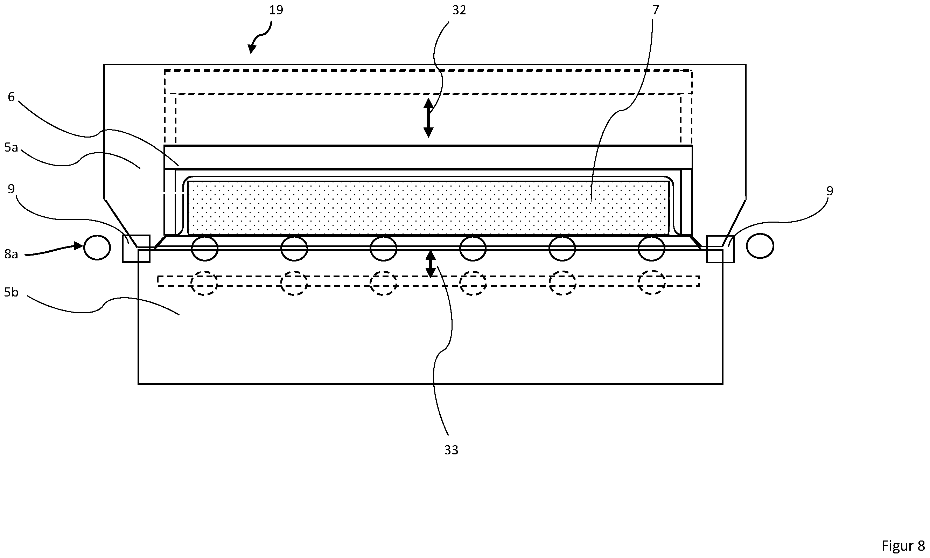

Mit der Figur 8 wird der Schweißprozess mit dem Folienschweißgerät 6 gezeigt. Hierfür ist das Folienschweißgerät 6 im Pfeilrichtung 32 nach unten gefahren, sodass es mit den beiden Folien 5a und 5b in Kontakt sind. Nachdem erfolgten Schweißvorgang findet ein Abschneiden der Restfolie 26 statt.

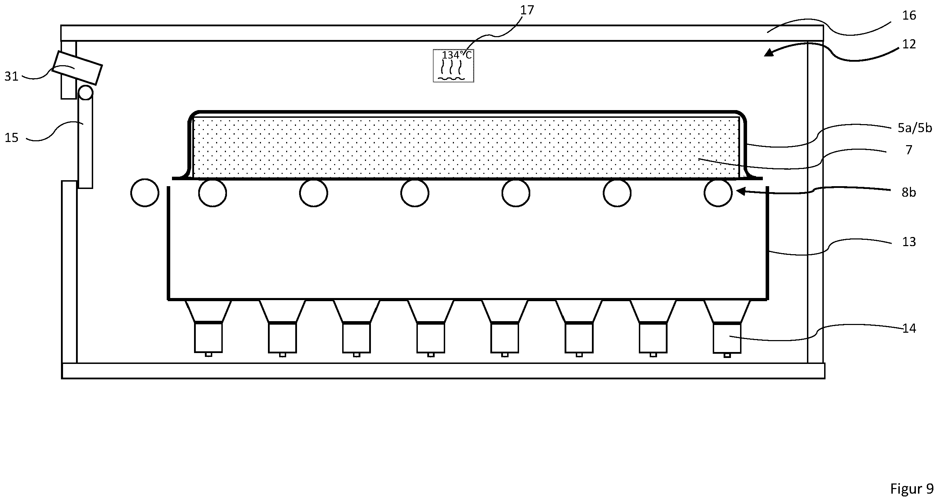

Figur 9 zeigt die Sterilisation des verpackten Trays 7 im gas- und druckdichten Hygieneraum 12.

### Zeichnungslegende

- 1.: Vorrichtung
- 2.: Klappe
- 3.: Bedienelemente
- 4.: Kamera
- 5.: Folie 5a (oben), Folie 5b (unten)
- 6.: Folienschweißgerät
- 7.: Tray
- 8.: Förderband
- 9.: Folienförderband
- 10.: Drucker
- 11.: Steuerung
- 12.: Hygieneraum
- 13.: Reinigungs- und Desinfektionsvorrichtung (z.B. Ultraschallbad)
- 14.: Ultraschallkopf
- 15.: Klappe
- 16.: Hygieneraumgehäuse
- 17.: Sterilisationsvorrichtung
- 18.: Gehäuse
- 19.: Auf- und Nachbereitungsvorrichtung
- 20.: Klappe
- 21.: Heizvorrichtung
- 22.: Pumpe
- 23.: Heißdampferzeuger
- 24.: UV-Lampen
- 25.: Technikraum
- 26.: Restfolie
- 27.: Wanne
- 28.: Pfeilrichtung von 13
- 29.: Tank
- 30.: Flüssigkeit für 13
- 31.: Druckluftquelle
- 32.: Pfeilrichtung von 6
- 33.: Pfeilrichtung von 8

## Patentansprüche

1. Vorrichtung (1) zur Aufbereitung von medizinischen Instrumenten, bestehend aus einem Hygieneraumgehäuse (16), in welchem eine Reinigungs- und Desinfektionsvorrichtung (13) angeordnet ist, welche als Ultraschallvorrichtung ausgebildet ist und eine Reinigung und Desinfektion der medizinischen Instrumente durchführt, **dadurch gekennzeichnet, dass** das Hygieneraumgehäuse (16) als ein gas- und druckdichtes Gehäuse ausgebildet ist, innerhalb welchem die Reinigungs- und Desinfektionsvorrichtung (13) und zusätzlich eine Sterilisationsvorrichtung (17) angeordnet ist, so dass innerhalb des Hygieneraumgehäuses (16) eine Reinigung, Desinfektion und Sterilisation der medizinischen Instrumente erfolgt.

2. Vorrichtung (1) nach Anspruch 1, **dadurch gekennzeichnet, dass** die Sterilisationsvorrichtung (17) als Autoklav ausgebildet ist, welcher eine thermische Sterilisation der medizinischen Instrumente im Überdruckbereich durchführt.

3. Vorrichtung (1) nach Anspruch 1 oder 2, **dadurch gekennzeichnet, dass** die Reinigungs- und Desinfektionsvorrichtung (13) vertikal bewegbar innerhalb des Hygieneraumgehäuse (16) angeordnet ist und durch die vertikale Bewegung ein Kontakt der Reinigungs- und Desinfektionsvorrichtung (13) mit den medizinischen Instrumenten erfolgt.

4. Vorrichtung (1) nach einem der Ansprüche 1 bis 3, **dadurch gekennzeichnet, dass** die Reinigungs- und Desinfektionsvorrichtung (13) eine Wanne (27) aufweist, an deren Bodenbereich mehrere Ultraschallköpfe (24) angeordnet sind, wobei sich in der Wanne (27) eine Flüssigkeit (30) zur Reinigung der medizinischen Instrumente befindet.

5. Vorrichtung (1) nach Anspruch 4, **dadurch gekennzeichnet, dass** die Flüssigkeit (30) in der Wanne (27) sowohl die medizinischen Instrumente als auch ein Förderband (8, 8a, 8b) im Bereich der Reinigungs- und Desinfektionsvorrichtung (13) bedeckt.

6. Vorrichtung (1) nach Anspruch 4 oder 5, **dadurch gekennzeichnet, dass** eine Heizvorrichtung (21) die Flüssigkeit (30) der Reinigungs- und Desinfektionsvorrichtung (13) zur thermischen Desinfektion auf mindestens 90 Grad erhitzt.

7. Vorrichtung (1) nach einem der Ansprüche 1 bis 6, **dadurch gekennzeichnet, dass** die vertikale Bewegung der Reinigungs- und Desinfektionsvorrichtung (13) mit einem Antrieb erfolgt, wobei der Antrieb als ein elektrischer Motor oder ein pneumatischer Antrieb ausgebildet ist.

8. Vorrichtung (1) zur Aufbereitung von medizinischen Instrumenten, bestehend aus in einem abgeschlossenen Gehäuse (18) mit mindestens einer Klappe (2), in welchem eine Reinigungs- und Desinfektionsvorrichtung (13) angeordnet ist, welche als Ultraschallvorrichtung ausgebildet ist und eine Reinigung und Desinfektion der medizinischen Instrumente durchführt, **dadurch gekennzeichnet, dass** innerhalb des abgeschlossenen Gehäuses (18) ein gas- und druckdichtes Hygieneraumgehäuse (16) angeordnet ist, in welchem sich die Reinigungs- und Desinfektionsvorrichtung (13) und zusätzlich eine Sterilisationsvorrichtung (17) befinden und dass sich in dem Gehäuse (18) eine Auf- und Nachbearbeitungsvorrichtung (19) für die medizinischen Instrumente befindet, die mindestens eine Folienverpackungsvorrichtung zum Verpacken der gereinigten medizinischen Instrumente aufweist.

9. Vorrichtung (1) nach Anspruch 8, **dadurch gekennzeichnet, dass** das Gehäuse (18) mindestens ein erstes Förderband (8, 8a) aufweist, mit welchem die medizinischen Instrumente im Bereich der Auf- und Nachbearbeitungsvorrichtung (19) bewegbar sind und mindestens ein damit verbundenes zweites Förderband (8, 8b), mit welchem die medizinischen Instrumente im Hygieneraumgehäuse (16) bewegbar sind.

10. Vorrichtung (1) nach Anspruch 8 oder 9, **dadurch gekennzeichnet, dass** die Auf- und Nachbearbeitungsvorrichtung (19) mindestens eine Kamera (4) zur bildlichen Erfassung der medizinischen Instrumente aufweist.

11. Vorrichtung (1) nach einem der Ansprüche 8 bis 10, **dadurch gekennzeichnet, dass** die Folienverpackungsvorrichtung ein Folienschweißgerät (6) und ein Folienförderband (9) aufweist, mit welchen eine erste Folien (5a) und eine zweite Folie (5b) um die medizinischen Instrumente gelegt werden und mit dem Folienschweißgeräte (6) verbunden werden.

12. Vorrichtung (1) nach einem der Ansprüche 8 bis 11, **dadurch gekennzeichnet, dass** mindestens eine UV-Lampe (24) eine Desinfektion innerhalb des Gehäuses (18), insbesondere im Bereich des Auf- und Nachbearbeitungsvorrichtung (19) durchführt.

13. Vorrichtung (1) nach einem der Ansprüche 8 bis 12, **dadurch gekennzeichnet, dass** dem Gehäuse (18) ein Technikraum (25) zugeordnet ist, in welchem sich eine Steuerung (11), ein Heißdampferzeuger (23), eine Heizvorrichtung (21), eine Pumpe (22) und ein Tank (29) befinden.

14. Vorrichtung (1) nach Anspruch 8 bis 13, **dadurch gekennzeichnet, dass** das Hygieneraum-Gehäuse (16) mindestens eine Druckluftquelle (31) aufweist, über welche Druckluft in das Gehäuse (16) einblasbar ist.

15. (ehemals 16.) Vorrichtung (1) nach einem der Ansprüche 8 bis 14, **dadurch gekennzeichnet, dass** die medizinischen Instrumente auf einem Förderband (8, 8a, 8b) von der Auf- und Nachbereitungsvorrichtung (19) in das Hygieneraumgehäuse (16) und zurück förderbar sind, wobei das Förderband (8, 8a, 8b) die medizinischen Instrumente im Hygieneraumgehäuse (16) über der Reinigungs- und Desinfektionsvorrichtung positioniert.
